# FASCICULE DE BREVET EUROPEEN

(11) **EP 1 827 382 B1**
(45) Date de publication et mention de la délivrance du brevet: **22.01.2014**
(21) Numéro de dépôt: 05850587.6
(22) Date de dépôt: 22.12.2005
(51) Int. Cl.: A61K 9/16

(54) **NOUVEAU SYSTEME GALENIQUE POUR LE TRANSPORT D'ACTIF, PROCEDE DE PREPARATION ET UTILISATION**
NEUES GALENISCHES SYSTEM FÜR DEN WIRKSTOFFTRANSPORT, HERSTELLUNGSVERFAHREN UND ANWENDUNG
NOVEL GALENIC SYSTEM FOR ACTIVE PRINCIPLE TRANSPORT, PREPARATION METHOD AND USE

(30) Priorité: 23.12.2004 FR 0413855
(43) Date de publication de la demande: 05.09.2007
(73) Titulaire: Capsugel France, 68000 Colmar (FR)
(72) Inventeur: IOUALALEN, Karim, F-31650 Saint Orens de Gameville (FR); RAYNAL, Rosanne, F-31650 Saint Orens De Gameville (FR)
(74) Mandataire: Hyden, Martin Douglas
(86) Numéro de dépôt international: PCT/FR2005/003247
(87) Numéro de publication internationale: WO 2006/070117

(56) Documents cités:
- WO-A-99/65448
- WO-A-2004/084856

## Description

La présente invention concerne un nouveau système galénique dont la stabilité est prolongée. Ledit système permet la protection d'un actif, particulièrement d'un médicament, contre sa dégradation lors du transit dans l'estomac lors d'une absorption par voie orale.

Le système galénique selon l'invention permet en outre le masquage du goût d'un actif éventuellement contenu dans le système galénique, la stabilisation dudit actif, la modulation des propriétés de libération dudit actif, le masquage des effets d'irritabilité mucosale et la toxicité de certains principes actifs.

La voie d'administration thérapeutique la plus simple et la plus pratique reste la voie orale. En France, elle constitue 75 % des prises médicamenteuses (Pharmacie galénique, A. Le Hir - Editions Masson.). Les formes galéniques destinées à la voie orale se présentent essentiellement sous deux formes, liquides et sèches. Elles présentent l'énorme avantage de ne pas nécessiter d'acte médical lors de la prise.

Le pH de l'estomac est compris entre 2 et 6. La nature acide du milieu stomacal peut entraîner la dégradation des actifs contenus dans les compositions ingérées, avant que ceux-ci n'atteignent l'intestin, où ils sont théoriquement absorbés au travers de la muqueuse intestinale pour passer dans la circulation. Un tel effet délétère du transit stomacal peut aller à l'encontre de l'objectif recherché à savoir l'absorption par l'organisme dudit actif dans sa forme la plus efficace pour l'effet recherché. Cet inconvénient est d'autant plus fort que l'on s'adresse aux compositions pharmaceutiques.

Il existe donc un besoin de véhicule pour les actifs ingérés par la voie orale, capable d'assurer audit actif un transit stomacal au cours duquel ledit actif ne sera pas dégradé. C'est un des buts de la présente invention.

D'autres problèmes sont bien connus avec les formes galéniques destinées à la voie orale, particulièrement les formes galéniques médicamenteuses. Un problème récurrent de ces formes galénique est l'observance.

L'observance est un facteur capital dont dépend directement l'efficacité du traitement thérapeutique. L'observance, on parle aussi de bon usage du médicament, est définie comme étant l'action de suivre un traitement médicamenteux conformément aux indications de la prescription : respect de la durée du traitement, du nombre et des horaires de prises. Pris à une dose ou une fréquence insuffisante, un médicament risque d'être inactif ou peu efficace. Dans le cas d'affections transitoires, une mauvaise observance du traitement ne fait que reculer le moment de la guérison et entraîne des rechutes, parfois responsables de graves complications. Dans le cas de maladies chroniques, une mauvaise observance peut être responsable de dommages irréversibles.

Les principales difficultés rencontrées lors de l'administration orale varient en fonction de la présentation.

Pour les formes sèches, cachets, gélules, capsules, les inconvénients sont la déglutition et le goût. Certaines populations comme les personnes âgées, les enfants, certaines personnes présentant des troubles mentaux, doivent s'orienter vers la forme liquide.

Pour les formes liquides, la prise est très facile, mais cette forme se heurte toujours à un problème non résolu de masquage du goût et d'instabilité de nombreux principes actifs en phase aqueuse.

C'est également un des objectif de la présente invention que de proposer un système galénique permettant un masquage du goût efficace.

Enfin, quelle que soit la forme, il existe également des problèmes d'irritabilité, de toxicité mucosale et de gastro-toxicité, rencontrés lors de la prise de certains actifs, particulièrement de médicaments comme les anti-inflammatoires.

C'est aussi un des but de la présente invention que de proposer un système galénique permettant une libération retardée d'un actif, particulièrement afin que celui-ci ne soit pas libéré dans l'estomac lors de l'ingestion. Cette propriété passe par l'obtention d'un système galénique stable en milieu acide, c'est-à-dire résistant à un pH acide.

Comme le rappelle le document PCT/US99/27981, page 2 ligne 4, les méthodes utilisées pour minimiser le mauvais goût sont variées, addition d'édulcorants, addition d'arômes, formulation effervescente et technologies d'enrobage. Seules les techniques d'enrobage tentent de masquer le goût alors que les autres approches essaient d'augmenter l'appétence de la préparation. Ces techniques d'enrobage ont aussi été retenues pour empêcher la libération des actifs gastrotoxique, dans l'estomac.

Les techniques d'enrobage consistent à mettre en place une couche de composés isolants, de polymères ou de mélanges, autour du principe actif pour l'isoler du milieu externe. De nombreux composés polymériques, naturels ou de synthèse ont été utilisés pour la constitution de cette couche externe. On distingue principalement les dérivés de cellulose comme l'hydroxypropylméthylcellulose .(HPMC), l'éthylcellulose, les carboxyméthylcelluloses, l'hydroxypropylméthylcellulose phtalate ou les mélanges de ces produits. Cette technique a donné des résultats intéressants pour la modulation de la vitesse de libération et pour la gastroprotection, mais l'homme de l'art sait que le masquage du goût n'est pas satisfaisant et la formulation dans l'eau reste instable dans le temps, ce qui est incompatible avec la préparation de formes aqueuses comme les sirops et les suspensions.

D'autres polymères ont été utilisés comme les dérivés de polyacrylates, les polymères amonio-méthacrylate ou le méthacrylate proposés par la société RÔHM, tel que décrit dans le document FR 2795962 et WO 98/47493. De nombreux travaux ont été réalisés avec l'amidon et surtout les polycarbophiles et Carbopol comme décrit dans le brevet WO 02/092106.

Ces techniques d'enrobage sont bien connues de l'homme de l'art. On peut distinguer les procédés d'enrobage physiques, basés sur la pulvérisation de la solution d'enrobage dans une turbine ou dans un lit fluidisé comme décrit dans les brevets WO 00/30617 et WO 02/092106 d'une part, et l'enrobage physicochimique basé sur la coacervation ou séparation de phases comme décrit dans le brevet US 3341416 d'autre part. Toutes ces techniques conduisent à la mise en place d'une ou plusieurs couches polymériques externes, recouvrant une particule centrale composée du principe actif pur ou d'un mélange sous forme de granulés de principe actif avec d'autres matériaux supports comme décrit dans le document EP1194125 de la société Prographarm.

Nous avons vu qu'il n'était pas possible de masquer le goût tout en conservant les propriétés d'absorption de la molécule initiale.

La libération immédiate au niveau du tube digestif, est conditionnée par l'utilisation de polymère pH dépendant très sensible au pH supérieur à 7 de la cavité buccale ou de l'estomac, ce qui implique l'addition d'acide à la formulation finale.

Ces technologies d'enrobage présentent un certain nombre d'inconvénients :
- la gastroprotection n'est pas totale
- le masquage du goût n'est pas total et dans le cas de composés à forte amertume, le goût est encore trop désagréable
- les cinétiques de libération sont modifiées
- les particules d'enrobage ont une taille de quelques centaines de microns, et sont perceptibles lors de l'absorption. Dans ce cas leur rupture peut entraîner un mauvais goût.
- les procédés d'enrobage sont complexes, comportent de nombreuses étapes et ont un coût élevé.

Ces technologies ne sont pas compatibles avec la préparation de sirops stables à long terme.

Ces technologies ne sont donc pas encore pleinement satisfaisantes.

Le document WO2004/084856 divulgue un système galénique, sous forme de particules lipidiques solides, strictement hydrophobe, ne contenant strictement pas d'eau, ni de tensioactifs, ni d'agents émulsionnants, ni de traces de solvants, comprenant au moins une cire hydrophobe et au moins un acide gras non neutralisé.

De façon totalement surprenante et inattendue, les inventeurs ont montré que l'addition d'un agent neutralisant l'actif incorporé aux compositions des particules lipidiques solides préparées suivant le procédé décrit dans le brevet WO 99/65448, contenant un principe actif, permet d'obtenir des particules hydrophobes stables dans l'estomac et qui se libèrent uniquement dans le tube digestif, autorisant ainsi une gastroprotection.

De plus, ces compositions permettent un masquage total du goût sans modifier les propriétés de libération de l'actif.

Ainsi la présente invention propose un nouveau système galénique permettant :
- la gastroprotection,
- le masquage du goût,
- la protection du principe actif, particulièrement en milieu acide,
- la possibilité de modulation des propriétés de libération,
- le masquage des effets d'irritabilité mucosale et de la toxicité de certains principes actifs,
- la préparation de formes aqueuses, à goût masqué, stables et pH indépendantes.

Le système galénique selon l'invention est caractérisé en ce qu'il est constitué par un mélange de composés hydrophobes insolubles dans l'eau, en ce qu'il est sous forme solide à température ambiante et qu'il est totalement dépourvu de composés tensioactifs, de résidus de solvant et d'eau pouvant être à l'origine de réactions d'hydrolyse ou d'oxydation d'un actif qu'il pourrait contenir. Ce système galénique a la capacité d'incorporer des composés de nature hydrophile, hydrophobe ou minérale.

Ainsi, l'invention a pour objet un système galénique, sous forme de particules lipidiques solides, strictement hydrophobes, ne contenant strictement pas d'eau, ni de tensioactifs, ni d'agents émulsionnants, ni de traces de solvants, caractérisé en ce qu'il comprend au moins un principe actif, au moins une cire hydrophobe et au moins un agent neutralisant l'actif incorporé tel que défini dans la revendication 1.

Par la suite dans le texte, les expressions "système galénique", "particule lipidique", voire encore "gouttelette" ou "gouttelette lipidique" s'entendront comme ayant la même signification.

Selon l'invention, l'agent neutralisant peut être un agent alcalinisant ou un agent acidifiant.

Selon une première forme de réalisation de l'invention, l'agent neutralisant est un agent acidifiant qui peut être choisi parmi l'acide citrique ou l'acide tartrique.

Sous cette première forme de réalisation, l'agent acidifiant est en une quantité qui peut être comprise entre 0,1.% et 20% en masse et préférentiellement entre 0,5% et 5%.

Selon une seconde forme de réalisation de l'invention, l'agent neutralisant est un agent alcalinisant, à savoir le bicarbonate de sodium.

Selon cette forme particulière de l'invention, l'agent alcalinisant peut être en une quantité comprise entre 0,1% et 30% en masse et préférentiellement entre 1% et 10%.

Selon une forme particulière de l'invention, le système galénique est en outre dépourvu de talc.

Selon encore une autre forme particulière de l'invention, les particules lipidiques sont solides à une température pouvant aller jusqu'à 45°C, préférentiellement jusqu'à 37,5°C.

Selon une autre forme particulière de l'invention, les particules lipidiques sont sous une forme sphérique.

Par cire hydrophobe selon l'invention, on entend que le système galénique peut être constitué d'une ou plusieurs cires, végétales, animales ou minérales, ou d'un mélange d'une ou plusieurs cires et d'au moins une huile non amphiphile.

Le système galénique peut en outre comprendre au moins un composé hydrophobe permettant d'ajuster le point de fusion et les propriétés physicochimiques comme la dureté. On peut citer à titre d'exemple de composé hydrophobe, la cire d'abeille ou encore l'huile de palme.

Il convient de choisir une composition appropriée, compatible en termes de toxicité, de biocompatibilité, de non immunogénicité et de biodégradabilité avec l'absorption par voie orale ou, tout autre mode d'administration. Dans ce cas, les composants seront choisis parmi les composants déjà utilisés pour l'administration par voie orale, tels que ceux définis dans la liste GRAS éditée par la "Food and Drug Administration", et de telle sorte que les particules formées conservent leurs propriétés d'incorporation, de masquage de goût et de stabilisation des composants actifs.

Ainsi selon l'invention, la cire peut être choisie parmi toute cire connue compatible avec les exigences de l'invention. La cire peut en particulier être choisie parmi
- les triglycérides et dérivés
- l'huile de palme
- la cire de Carnauba
- la cire de Candellila
- la cire d'Alfa
- le beurre de cacao
- l'ozokérite
- les cires végétales comme la cire d'olivier, la cire de riz, la cire de jojoba hydrogénée ou les cires absolues de fleurs
- les cires d'abeilles et cires d'abeilles modifiées.

Le selon une forme particulière de l'invention, la cire peut être un mélange de cires.

Selon l'invention, il est possible d'utiliser une cire ou un mélange de cires, dont le point de fusion peut être compris entre 15°C et 70°C, préférentiellement entre 30°C et 45°C.

Selon l'invention, la cire peut être en une quantité comprise entre 0,5% et 99%, préférentiellement entre et 1% et 50%

Le système galénique selon l'invention peut en outre contenir des additifs huileux, pâteux ou solides, des ingrédients actifs liposolubles ou hydrosolubles.

Il est possible d'utiliser d'autres composés, comme les alcools gras de haut poids moléculaire, les acides gras préférentiellement linéaires et saturés pairs ayant de 12 à 30 atomes de carbone, les esters d'acides et d'alcools à haut poids moléculaire notamment les esters des acides linéaires et saturés pairs ayant de 4 à 20 atomes de carbone et des alcools linéaires et saturés pairs ayant de 14 à 32 atomes de carbone. Dans tous les cas le mélange obtenu doit être caractérisé par un point de fusion final compris entre 15°C et 70°C, par l'absence de composés tensioactifs, par un comportement hydrophobe et une non mouillabilité par l'eau. Outre les cires mentionnées ci-dessus, la composition selon l'invention peut contenir une huile ou un mélange choisis parmi :
- les huiles de silicones hydrophobes de viscosité comprise entre 5 et 9000 centistockes, les cyclométhicones,
- les huiles organofluorées lipophiles,
- le perhydrosqualène.

D'autres composés huileux comme l'alcool oléique, la lanoline, l'huile de tournesol, l'huile de palme, l'huile d'olive, les acides gras et alcools gras peuvent être utilisés, mais le mélange huileux obtenu doit être caractérisé par un comportement hydrophobe, une absence de miscibilité avec l'eau et un point de fusion compris entre 15°C et 70°C, préférentiellement compris entre 30°C et 45°C.

Il est en outre possible pour ajuster la consistance, d'introduire dans la composition des argiles ou leurs dispersions huileuses, des gommes de silicones phénylées, des amidons, des structurants des corps gras.

On peut ajouter à la matrice hydrophobe du système galénique, un certain nombre de composés comme des charges minérales permettant de moduler la densité et la plasticité. Parmi les composés minéraux, on choisira de façon avantageuse le talc, le kaolin.

Les particules lipidiques selon l'invention peuvent avoir une taille comprise entre 0,5 microns et 1500 microns, préférentiellement entre 10 microns et 250 microns.

Les particules lipidiques selon l'invention présentent l'avantage de permettre la libération retardée d'un actif qu'elles pourraient contenir, une très grande stabilité à pH acide, particulièrement en formulation aqueuse acide, permettant ainsi la protection de l'actif lorsqu'elles sont en contact avec un milieu présentant un pH acide comme par exemple le milieu gastrique, et un masquage total du goût.

Dans cette description, le terme actif est utilisé pour désigner n'importe quelle substance utilisable en cosmétique, en pharmacie, en biotechnologie, dans le domaine vétérinaire ou encore en alimentation. Particulièrement selon l'invention, l'actif peut être une substance thérapeutique active pouvant être avantageusement administré à l'homme ou aux autres animaux pour diagnostiquer, soigner, réduire, traiter ou prévenir la maladie.

Selon l'invention, l'actif peut être tout composé de nature hydrophile, hydrophobe ou minérale.

Selon l'invention, l'actif peut être dissous ou dispersés dans le système galénique.

Bien entendu selon l'invention, l'actif peut être un mélange d'actifs.

L'actif peut être choisi parmi des huiles essentielles, des arômes, des pigments, des charges, des colorants, des enzymes et coenzymes et d'autres substances actives.

Parmi les actifs pouvant être incorporés dans le système galénique selon l'invention, on peut citer les vitamines ou provitamines A, B, C, D, E, PP et leurs esters, les caroténoïdes, les substances anti-radicalaires, les hydroxyacides, les antiseptiques, les molécules agissant sur la pigmentation, sur l'inflammation, les extraits biologiques.

L'actif peut aussi être choisi parmi des conservateurs, des antioxydants, des colorants et pigments, des cellules et organites cellulaires ou encore des composants pharmaceutiques destinés à traiter des pathologies, particulièrement des pathologies cutanées ou mucosales.

A titre d'exemple d'actif thérapeutique pouvant être incorporé dans le système galénique selon l'invention, on peut citer les antibiotiques, les antitussifs comme le dextrométhorphane et ses dérivés ou la codéïne, les antifongiques dont les composés azolés comme le kétoconazole, les antiparasitaires dont l'amphotéricine B et les dérivés benzimidazolés, les antipaludéens dont le proguanil, la chloroquine, les adsorbants, les hormones et dérivés, la nicotine, les antihistaminiques, les corticoïdes, les anti-inflammatoires stéroïdiens et non stéroïdiens dont les arycarboxyliques, l'ibuprofène et le kétoprophène, les agents antiallergiques, les antalgiques dont le paracétamol et les composés salycilés, la Noramidopyridine et ses dérivés, les anesthésiques locaux, les antalgiques, les antiviraux, les anticorps, les molécules agissant sur le système immunitaire comme la cyclosporine et le tacrolimus, les cytostatiques et anticancéreux dont les alkylants, les nitroso-urées, les organoplatines, les taxoïdes et leurs dérivés, les vinca-alcaloïdes, les hypolipémiants dont les statines et les fibrates, les vasodilatateurs, les vasoconstricteurs, les inhibiteurs de l'enzyme de conversion de l'angiotensine et de la phosphodiestérase, les dérivés nitrés et antiangoreux, les antiarythmie dont le diltiazem, les béta-bloquants, les inhibiteurs calciques, les antidiurétiques et diurétiques, les bronchodilatateurs, les opiacés et dérivés, les barbituriques, les benzodiazépines, les molécules agissant sur le système nerveux central, les acides nucléiques, les peptides dont l'insuline et la calcitonine, les composés anthracéniques, l'huile de paraffine, le polyéthylène glycol, les sels minéraux, les antispasmodiques, les antisécrétoires gastriques en particulier la classe des benzimidazols dont l'Oméprazole, le Lansoprazole l'ésoméprazole et leurs dérivés, les pansements gastriques argiles et polyvinylpyrrolidone, l'amidon. Cette liste exhaustive n'est en aucun cas limitative.

Selon l'invention, les particules lipidiques ont une température de fusion, après incorporation du principe actif, comprise entre 15°C et 70°C, préférentiellement comprise entre 30°C et 45°C.

La capacité de chargement des particules en actif peut s'étendre de 0,02% à 75% par rapport au poids de particules, particulièrement de 5% à 50%.

Selon encore un autre mode de réalisation de l'invention, il est possible d'introduire dans le système galénique selon l'invention un acide gras non neutralisé qui peut être tout acide gras non neutralisé compatible avec les exigences de l'invention. L'acide gras non neutralisé peut être choisi parmi les acides gras à chaînes linéaires ayant un nombre d'atomes de carbone compris entre 4 et 18, tels que par exemple l'acide myristique, l'acide laurique, l'acide palmitique, l'acide béhénique ou encore l'acide oléique.

Selon un mode de réalisation particulier de l'invention, l'acide gras non neutralisé peut être constitué d'un mélange d'acides gras non neutralisé

Selon l'invention, l'acide gras non neutralisé peut être utilisé à un taux d'acides gras compris entre 0,5% et 75% en masse et préférentiellement entre 1% et 30%.

L'homme de l'art sait que lorsqu'on effectue l'incorporation de ces actifs dans le système galénique, il convient de choisir une composition lipidique appropriée de telle sorte que les particules soient solides à la température d'utilisation avec une taille comprise de préférence entre 0,5 microns et 1500 microns et de préférence entre 0,5 microns et 100 microns, permettent d'obtenir un masquage total du goût sans modifier les propriétés de libération et en présentant une très grande stabilité en formulation aqueuse même à pH élevé. En outre, il est nécessaire que le procédé de préparation dudit système galénique comprenant en outre un actif puisse être mis en oeuvre.

Selon l'invention, le système galénique comprenant en outre un actif peut être préparé suivant le procédé décrit dans le brevet WO 99/65448.

Selon ce mode de réalisation du procédé, les particules sont obtenues par mélange à chauffage modéré. Plus précisément dans une première étape, la cire, et éventuellement l'acide gras non neutralisé, est portée sous agitation, à une température à 2°C ou 3°C au dessus du point de fusion du composé présentant le point de fusion le plus élevé, et mélangée ; dans une deuxième étape, on ajoute l'agent neutralisant et l'actif en maintenant une agitation intense ; dans une troisième étape, on forme des gouttelettes lipidiques comprenant l'actif en dispersant le mélange obtenu à la première étape dans un gel avec lequel ledit mélange est non-miscible, préalablement porté à la même température que le mélange obtenu à la première étape, et de concentration en agent gélifiant comprise entre 0,1 g/l et 30 g/l, de préférence entre 0,2 g/l et 20 g/l ; dans une quatrième étape, dès la fin de l'injection, les gouttelettes sont refroidies immédiatement sous la température de solidification du mélange, puis lavées avec de l'eau, pouvant éventuellement contenir de l'éthanol ; dans une cinquième étape, les particules lavées sont récupérées par tamisage puis séchées.

En comparaison avec les techniques de fusion chaude, le procédé de la présente invention, ne fait pas intervenir d'agents émulsionnants ni de produits amphiphiles dans la composition, pour permettre une dispersion stable lors de la phase de solidification par refroidissement.

Selon un mode de réalisation particulier du procédé selon l'invention, quand il est nécessaire d'assurer une élimination totale de l'actif de la surface des particules lipidiques, celui-ci comprend une étape de lavage desdites particules obtenues avec un mélange de lavage comprenant de l'éthanol. Dans ce cas, la présence d'éthanol dans le mélange de lavage est essentielle au procédé car l'éthanol permet un lavage complet des éventuels résidus d'actif pouvant être présents à la surface des particules lipidiques qui pourraient générer un goût désagréable. Ainsi selon cette mise en oeuvre particulière du procédé selon l'invention, le mélange des différents composants du système galénique (la cire et l'acide gras non neutralisé entre autres) et de l'actif est réalisé dans une première étape du procédé. Ce mélange est réalisé à chaud à 2°C ou 3°C au-dessus du point de fusion du composé présentant le point de fusion le plus élevé. L'homme de l'art sait qu'il est nécessaire d'appliquer un mode d'agitation approprié à la dispersion de tous les composants.

Puis dans une deuxième étape on forme des gouttelettes lipidiques comprenant l'actif en dispersant le mélange obtenu à la première étape dans un gel préparé avec un agent gélifiant, rhéofluidifiant et non tensioactif, avec lequel ledit mélange est non miscible, préalablement porté à la même température, et de concentration en agent gélifiant comprise entre 0,1g/l et 30g/l, préférentiellement comprise entre 0,2g/l et 20g/l suffisamment élevée pour figer la dispersion.

Il peut être préférable d'injecter la composition au sein du gel, par exemple par un orifice situé à la base d'un réacteur. L'agitation qui doit être maintenue tout au long de l'injection a pour caractéristique de présenter une pale équipée d'une ancre, destinée à disperser la composition et une deuxième pale axiale équipée d'une hélice tripale destinée à former les gouttelettes de dispersion de taille souhaitée. Cette dernière étape est extrêmement rapide puisque les gouttelettes sont obtenues au fur et à mesure de l'injection de la composition. L'agitation n'a pas besoin d'être maintenue après la fin de l'injection, car les gouttelettes sont figées dans le gel.

Dans une troisième étape du procédé, dès la fin de l'injection, les gouttelettes sont refroidies immédiatement, sous la température de solidification du mélange, puis lavées.

La phase de lavage est très importante car elle permet de ne pas avoir de résidus à la surface des particules, qui pourraient générer un goût désagréable.

Ainsi, quelle que soit la forme de mise en oeuvre du procédé de l'invention, le lavage des particules peut être réalisé en utilisant un mélange de lavage constitué d'eau et d'éthanol en une proportion comprise entre 0% et 25% d'éthanol.

Enfin dans une quatrième étape, les particules lavées sont ensuite récupérées par tamisage puis séchées. Les particules obtenues présentent une grande homogénéité de taille et peuvent être manipulées industriellement sans précaution particulière.

L'homme de l'art sait qu'on peut aussi utiliser d'autres modes de dispersion comme la sonication ou les mélangeurs statiques.

Le procédé selon l'invention est donc rapide et ne nécessite pas d'étape d'agitation longue et délicate. Il permet d'incorporer l'actif au système galénique dès la première étape du mélange des différents ingrédients de la composition.

Parmi les agents gélifiants rhéofluidifiants et non tensioactifs appropriés pour la formation du gel utilisé comme milieu de dispersion selon le procédé, on peut citer les polymères carboxyvinyliques tels que les polymères polyacryliques non modifiés par des groupements hydrophobes ou surfactants, les carraghénanes, les épaississants et gélifiants polysaccharidiques comme les xanthanes, les gommes de guar et de caroube, les alginates, les dérivés de cellulose, les pectines, l'agar, ou un mélange de ceux-ci.

Les particules lipidiques comprenant un actif peuvent être incorporées dans toute composition, particulièrement dans toute composition, cosmétique, pharmaceutique, vétérinaire ou alimentaire.

Ainsi l'invention a aussi pour objet une composition comprenant au moins une particule lipidique contenant un actif.

La composition selon l'invention, peut en outre comprendre tout additif visant à en modifier l'aspect ou la rhéologie.

Par exemple, à la poudre sèche de particules, on peut ajouter des agents lubrifiants qui améliorent la fluidité des particules comme le talc, les amidons, les poudres de silice, les agents antistatiques.

Bien entendu la composition selon l'invention peut être sous la forme de toute formulation galénique adéquate.

Dans une mise en forme avantageuse de réalisation, les particules de l'invention sont mises en oeuvre au sein de suspensions aqueuses, sirops et sachets.

Enfin les particules peuvent être mises en oeuvre au sein de formulations galéniques classiques du type capsules, gélules, granulés, poudres orales, poudres dispersibles, comprimés, comprimés hydrodispersibles et orodispersibles.

Selon un autre aspect de l'invention, les compositions peuvent être utilisées pour l'administration par voie injectable et en particulier pour la préparation de formes implantables à libération prolongée.

Dans ce cas, les particules lipidiques selon l'invention, sont préparées pour avoir une taille comprise préférentiellement entre 0,5 µm et 5 µm. Il est préférable de les tamiser pour obtenir une distribution de taille conforme avec le mode d'administration.

Leur composition cireuse est choisie pour être conforme avec les nécessités de la voie injectable.

Cette forme galénique permet d'éliminer les problèmes de toxicité rencontrés par les particules polymériques obtenues avec les procédés de polymérisation en émulsion, liés à l'utilisation des solvants et des composés tensioactifs.

Les particules selon l'invention permettent d'obtenir des taux de chargement en principe actif compris entre 0,10 et 3 grammes/gramme de matrice cireuse.

L'homme de l'art sait que les technologies d'encapsulation ne permettent pas d'atteindre ces taux. Enfin la dégradation des particules n'entraîne pas de réaction inflammatoire comme on peut le rencontrer avec les particules injectables à base de polymère de polylactique-glycolique.

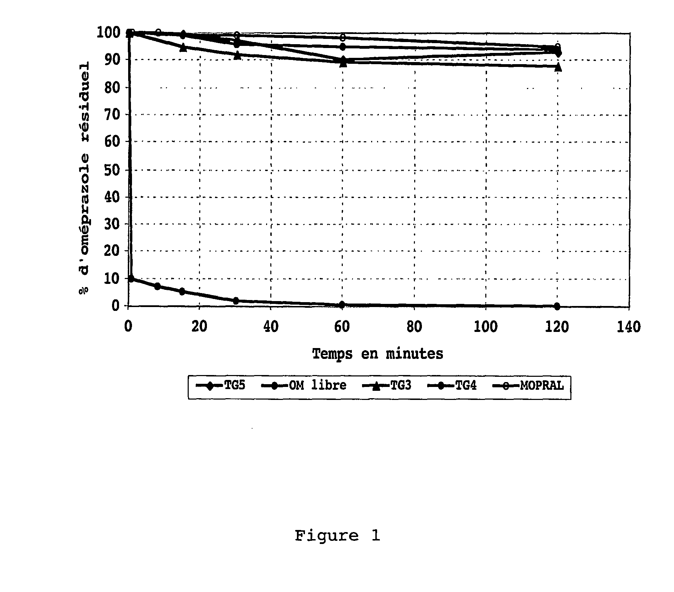

La figure 1 montre les résultats obtenus dans le test de résistance en milieu acide d'Oméprazole libre (OM libre) ou sous forme de particules selon l'invention (TG3, TG4 comparé à la forme pharmaceutique (MOPRAL®).

Les exemples qui suivent ne sont pas limitatifs, ils servent seulement à illustrer l'invention.

### Exemple 1 : Préparation de particules contenant de l'Oméprazole de point de fusion de 35°C

Exemple donné pour la fabrication de 100g de particules contenant de l'Oméprazole :
Composition TG1 :

| | |
|---|---|
| Mélange de Triglycérides saturés de point de fusion 35°C (Captex® de la société ABITEC) | 79 g |
| Bicarbonate de sodium : | 1 g |
| Oméprazole : | 20 g |

### Mode opératoire pour la préparation des particules :

Dans un récipient thermostaté, on porte le composé de plus haut point de fusion, 2°C au-dessus de sa température de fusion, puis on ajoute progressivement les différents composés du point de fusion le plus élevé au moins élevé.

La température du mélange est progressivement abaissée pour être maintenue à 5°C au-dessus de la température de fusion du nouveau mélange obtenu.

On ajoute en dernier l'Oméprazole. La dispersion de ces composants dans la phase lipidique, est réalisée à l'aide d'un système d'agitation de type turbine de marque Turrax à une vitesse de 10000 tours/min .tours/min le mélange est homogène, il est ajouté à 600 ml de gel aqueux rhéofluidifiant à 0,2% de carbopol Ultrez 10, neutralisé à pH 7 ± 1 avec de la soude, préalablement porté à la même température que le mélange lipidique et contenu dans un réacteur équipé d'un système d'agitation à hélice tripale.

Pendant l'addition de la composition, la vitesse d'agitation de l'hélice tripale est de 180 tours/min ± 90.

L'agitation est maintenue pendant 30 secondes après la fin de l'addition de la composition, puis stoppée.

La dispersion est alors refroidie à 15°C.

Les particules sont récupérées par tamisage puis lavées à l'eau purifiée, puis récupérées et séchées.

Les particules ainsi obtenues ont une taille moyenne de 230 microns.

### Exemple 2 : Préparation de particules contenant de l'Oméprazole de point de fusion de 35°C sans agent alcalinisant

Exemple donné pour la fabrication de 100 g de particules.

### Composition TG2 :

| | |
|---|---|
| Mélange de Triglycérides saturés de point de fusion 35°C (Captex® de la société ABITEC) | 80 g |
| Oméprazole : | 20 g |

Les particules sont préparées selon le protocole décrit dans l'exemple 1.

### Exemple 3 : Préparation de particules contenant de l'Oméprazole de point de fusion égale à 38°C

Exemple donné pour la fabrication de 100 g de particules.

### Composition TG3 :

| | |
|---|---|
| Triglycérides d'acide caprique, caprylique et laurique de point de fusion 38° | 79 g |
| Bicarbonate de sodium : | 1 g |
| Oméprazole : | 20 g |

Les particules sont préparées selon le protocole décrit dans l'exemple 1

### Exemple 4 : Préparation de particules contenant de l'Oméprazole, de point de fusion égale à 42°C

Exemple donné pour la fabrication de 100 g de particules.

### Composition TG4 :

| | |
|---|---|
| Triglycérides d'acide caprique, caprylique et laurique de point de fusion 38°C : | 75,05 g |
| Paraffine | 3,95 g |
| Bicarbonate de sodium : | 1,00 g |
| Oméprazole : | 20,00 g |

Les particules sont préparées selon le protocole décrit dans l'exemple 1

### Exemple 5 : Préparation de particules contenant de l'Oméprazole, de point de fusion égale à 42°C, sans agent alcalinisant.

Exemple donné pour la fabrication de 100 g de particules.

### Composition TG5 :

| | |
|---|---|
| Triglycérides d'acide caprique, caprylique et laurique de point de fusion 38°C | 76 g |
| Paraffine | 4 g |
| Oméprazole | 20 g |

Les particules sont préparées selon le protocole décrit dans l'exemple 1

### Exemple 6 : Préparation de particules contenant du chlorhydrate d'Oxytétracycline de point de fusion égale à 38°C.

Exemple donné pour la fabrication de 100 g de particules.

### Composition TG6 :

| | |
|---|---|
| Triglycérides d'acide caprique, caprylique et laurique de point de fusion 38°C | 79 g |
| acide tartrique micronisé : | 1 g |
| Chlorhydrate d'oxytetracycline : | 20 g |

Les particules sont préparées selon le protocole décrit dans l'exemple 1.

### Exemple 7 : comparaison de la résistance en milieu acide et de la stabilité à long terme de particules d'Oméprazole contenant ou pas d'agent alcalinisant ou neutralisant les acides.

### Etude de résistance en milieu acide

Pour déterminer la résistance au pH acide, les préparations sont placées en milieu agité à pH 1,2 à 37,5°C. La résistance au pH acide est déterminée par le dosage du taux d'Oméprazole après neutralisation à pH=6,8 en fonction du temps.

Les résultats rassemblés dans la figure n°1, montrent que les formulations d'Oméprazole selon l'invention ainsi que les granules de MOPRAL® sont résistantes au pH acide. L'Oméprazole libre est dégradé en quelques minutes. L'Oméprazole est dosée par HPLC après extraction.

### Etude de stabilité

Etude de stabilité de microparticules d'Oméprazole préparées à partir des formulations selon les exemples précédents. La stabilité est évaluée par la détermination du taux d'impuretés.

**Tableau 1**

| Exemple | Bicarbonate de sodium | | T=0 | 1 mois | 2 mois | 3 mois | 6 mois |
|---|---|---|---|---|---|---|---|
| TG1 | 1% | I | 0 | 0 | 0 | 0,01 | 0,02 |
| | | C | B1 | bl | bl | bl | B1 |
| TG2 | 0 | I | 0 | 0 | 0,01 | 0,03 | 0,07 |
| | | C | Bl | bl | bl | bl | Be |
| TG4 | 1% | I | 0 | 0 | 0 | 0,02 | 0,03 |
| | | C | Bl | bl | bl | bl | Bl |
| TG5 | 0 | I | 0 | 0 | 0,01 | 0,03 | 0,06 |
| | | C | Bl | bl | Bl | Be | Be |

| | | | | | | | |
|---|---|---|---|---|---|---|---|
| I = impureté ; C = couleur ; Bl = blanchâtre ; Be = Beige | | | | | | | |

Les impuretés sont déterminées conformément à la pharmacopée Européenne.

**Tableau 2**

| Formulation | Bicarbonate de sodium | Teneur en Oméprazole en (mg/100 mg) | | | |
|---|---|---|---|---|---|
| | | T=0 | 1 mois | 3 mois | 6 mois |
| TG1 | 1% | 20,1 | 20,0 | 20,1 | 20,0 |
| TG2 | 0 | 20,0 | 19,9 | 19,9 | 19,7 |
| TG4 | 1% | 19,9 | 20 | 19,9 | 19,8 |
| TG5 | 0 | 20,1 | 20,2 | 20,0 | |

Le taux d'Oméprazole est suivi en fonction du temps par HPLC.

Les résultats des tableaux n° 2 et n° 3 montrent que les compositions contenant les agents alcalinisants présentent une stabilité plus élevée.

### Exemple 8 : Evolution au cours du temps de la concentration plasmatique d'Oméprazole administrée chez le rat pour différentes formulations.

Ces essais sont destinés à vérifier que les microparticules, selon l'invention, n'induisent pas de diminution de la biodisponibilité.

L'étude est réalisée en comparant les principaux paramètres de biodisponibilité des différentes particules selon l'invention contenant de l'Oméprazole, contre des microgranules d'une forme antérieure commercialisée par la société Astra sous le nom de MOPRAL®.

Les valeurs des principaux paramètres de biodisponibilités, pour les différentes formulations d'Oméprazole, sont rassemblées dans le tableau n°3.

**Tableau 3**

| Tests | Voie D'administration | Cmax | Tmax | AUC | AUC test/AUC Réf |
|---|---|---|---|---|---|
| | | Ng/ml | h | ng.h/ml | |
| Particule TG1 | PO | 124,1 | 0,1 | 95,1 | 221 |
| Particule TG2 | PO | 120,1 | 0,1 | 92,1 | 212 |
| Particule TG3 | PO | 211,0 | 0,12 | 115,6 | 265 |
| Particule TG4 | PO | 210,4 | 0,11 | 114,5 | 265 |
| Microgranules de MOPRAL® | PO | 14,33 | 2,25 | 43,52 | - |

| | | | | | |
|---|---|---|---|---|---|
| - AUC = Aire sous la courbe de la concentration plasmatique d'Oméprazole en fonction du temps - La voie PO est la voie orale - Cmax : Concentration plasmatique maximale - Tmax : durée nécessaire pour obtenir la Cmax) | | | | | |

L'étude des paramètres de biodisponibilité est menée sur des rats de type Sprague Dawley. Les résultats montrent que les particules, selon l'invention, n'induisent pas de diminution de la biodisponibilité.

### Exemple 9 : Préparation de particules contenant de Lansoprazole.

Exemple donné pour la fabrication de 100 g de particules.

### Composition

| | |
|---|---|
| Triglycérides d'acide caprique et caprylique : | 70,0 g |
| Triglycérides d'acide caprique et laurique | 9,5 g |
| Bicarbonate de sodium : | 0,5 g |
| Lansoprazole : | 20,0 g |

Les particules sont préparées selon le protocole décrit dans l'exemple 1

### Exemple 9 : Particules contenant de l'Erythromycine

Exemple donné pour la fabrication de 120 g de particules contenant de l'Erythromycine :
Composition :

| | |
|---|---|
| Triglycérides d'acide caprique et caprylique : | 73 g |
| Triglycérides d'acide caprique et laurique : | 10 g |
| Trilaurine | 5 g |
| Bicarbonate de sodium et phosphate de sodium (80/20) | 2 g |
| Erythromycine | 30 g |

soit 120 g de particules sèches contiennent 30 g d'érythromycine

## Revendications

1. Utilisation d'un système galénique pour la protection d'un actif contre sa dégradation lors du transit dans l'estomac lors d'une absorption par voie orale, **caractérisé en ce que** le système galénique, sous forme de particules lipidiques solides, strictement hydrophobe, ne contenant strictement pas d'eau, ni de tensioactifs, ni d'agents émulsionnants, ni de traces de solvants, comprend au moins un principe actif, au moins une cire hydrophobe et au moins un agent neutralisant l'actif incorporé ; et **caractérisé en ce que** l'agent neutralisant est le bicarbonate de sodium en tant qu'agent alcalinisant ou un agent acidifiant choisi parmi l'acide citrique et l'acide tartrique.

2. Utilisation d'un système galénique selon la revendication 1, **caractérisé en ce que** l'agent acidifiant est en une quantité comprise entre 0, 1 % et 20% en masse et préférentiellement entre 0,5% et 5%.

3. Utilisation d'un système galénique selon la revendication 1, **caractérisé en ce que** le bicarbonate de sodium est en une quantité comprise entre 0,1 % et 30% en masse et préférentiellement entre 1% et 10%.

4. Utilisation d'un système galénique selon l'une quelconque des revendications 1 à 3, **caractérisé en ce que** la cire hydrophobe est une cire végétale, animale ou minérale, ou un mélange d'au moins une cire et d'au moins une huile non amphiphile.

5. Utilisation d'un système galénique selon l'une quelconque des revendications 1 à 4, **caractérisé en ce que** la cire à un point de fusion qui peut être compris entre 15°C et 75°C, préférentiellement entre 30°C et 45°C.

6. Utilisation d'un système galénique selon l'une quelconque des revendications 1 à 5, **caractérisé en ce que** la cire est en une quantité comprise entre 0,5% et 99%, préférentiellement entre 1% et 55%.

7. Utilisation d'un système galénique selon l'une quelconque des revendications 1 à 6, **caractérisé en ce que** capacité de chargement des particules en actif peut s'étendre de 0,02 % à 75 % par rapport au poids de particules, particulièrement de 5% à 50%.

8. Utilisation d'un système galénique selon l'une quelconque des revendications 1 à 7, **caractérisé en ce que** l'actif est choisi parmi les vitamines ou provitamines A, B, C, D, E, PP et leurs esters, les caroténoïdes, les substances anti-radicalaires, les hydroxyacides, les antiseptiques, les molécules agissant sur la pigmentation, sur l'inflammation, les extraits biologiques, les antibiotiques, les antifongiques, les antiparasitaires, les antipaludéens, les adsorbants, les hormones et dérivés, la nicotine, les antihistaminiques, les anti-inflammatoires stéroïdiens et non stéroïdiens, les composés salicylés, les agents antiallergiques, les antalgiques, les anesthésiques locaux, les antiviraux, les anticorps et molécules agissant sur le système immunitaire comme la cyclosporine, le tacrolimus, les hypolipémiants comme les fibrates et les statines, les cytostatiques et anticancéreux, les antalgiques, les vasodilatateurs, les vasoconstricteurs, les inhibiteurs de l'enzyme de conversion de l'angiotensine et de la phosphodiestérase, les dérivés nitrés et antiangoreux, les béta bloquants, les inhibiteurs calciques, les antidiurétiques et diurétiques, les bronchodilatateurs, les opiacés et dérivés, les barbituriques, les benzodiazépines, les molécules agissant sur le système nerveux central, les acides nucléiques, les peptides, les composés anthracéniques, l'huile de paraffine, le polyéthylène glycol, les sels minéraux, les antispasmodiques, les antisécrétoires gastriques, en particulier la classe des benzimidazols dont l'Oméprazole, le Lansoprazole et l'ésoméprazole, les pansements gastriques argiles et polyvinylpyrrolidone, l'amidon.

9. Utilisation d'un système galénique selon l'une quelconque des revendications 1 à 8, **caractérisé en ce qu'**il est solide à une température pouvant aller jusqu'à 45°C, préférentiellement jusqu'à 37,5°C.

10. Utilisation d'un système galénique selon l'une quelconque des revendications 1 à 9, **caractérisé en ce que** les particules lipidiques sont sous une forme sphérique.

11. Utilisation d'un système galénique selon l'une quelconque des revendications 1 à 10, **caractérisé en ce qu'**il se présente sous la forme de particules lipidiques ayant une taille comprise entre 0,5 microns et 1500 microns, préférentiellement entre 10 microns et 250 microns.

## Patentansprüche

1. Verwendung eines galenischen Systems zum Schutz eines Wirkstoffs gegen dessen Abbau beim Transit durch den Magen bei einer Absorption auf oralem Weg, **dadurch gekennzeichnet, dass** das galenische System, das die Form von festen Lipidpartikeln aufweist, streng hydrophob ist, streng weder Wasser, noch Tenside, noch Emulgatoren, noch Spuren von Lösemitteln enthält, mindestens einen Wirkstoff, mindestens ein hydrophobes Wachs und mindestens ein Mittel, das den integrierten Wirkstoff neutralisiert, umfasst; und **dadurch gekennzeichnet, dass** das neutralisierende Mittel Natriumbicarbonat als alkalisierendes Mittel ist, oder ein säurebildendes Mittel, ausgewählt aus Citronensäure und Weinsäure.

2. Verwendung eines galenischen Systems nach Anspruch 1, **dadurch gekennzeichnet, dass** das säurebildende Mittel in einer Menge im Bereich zwischen 0,1 Gew.-% und 20 Gew.-% und vorzugsweise zwischen 0,5 Gew.-% und 5 Gew.-% vorhanden ist.

3. Verwendung eines galenischen Systems nach Anspruch 1, **dadurch gekennzeichnet, dass** das Natriumbicarbonat in einer Menge im Bereich zwischen 0,1 Gew.-% und 30 Gew.-% und vorzugsweise zwischen 1 Gew.-% und 10 Gew.-% vorhanden ist.

4. Verwendung eines galenischen Systems nach einem der Ansprüche 1 bis 3, **dadurch gekennzeichnet, dass** das hydrophobe Wachs ein pflanzliches, tierisches oder mineralisches Wachs oder ein Gemisch aus mindestens einem Wachs und mindestens einem nicht amphiphilen Öl ist.

5. Verwendung eines galenischen Systems nach einem der Ansprüche 1 bis 4, **dadurch gekennzeichnet, dass** das Wachs einen Schmelzpunkt aufweist, der im Bereich zwischen 15 °C und 75 °C, vorzugsweise zwischen 30 °C und 45 °C liegen kann.

6. Verwendung eines galenischen Systems nach einem der Ansprüche 1 bis 5, **dadurch gekennzeichnet, dass** das Wachs in einer Menge im Bereich zwischen 0,5 % und 99 %, vorzugsweise zwischen 1 % und 55 % vorhanden ist.

7. Verwendung eines galenischen Systems nach einem der Ansprüche 1 bis 6, **dadurch gekennzeichnet, dass** sich die Wirkstoffbeladungskapazität der Partikel, bezogen auf das Gewicht der Partikel, von 0,02 % bis 75 %, insbesondere von 5 % bis 50 % erstrecken kann.

8. Verwendung eines galenischen Systems nach einem der Ansprüche 1 bis 7, **dadurch gekennzeichnet, dass** der Wirkstoff ausgewählt ist aus den Vitaminen oder Provitaminen A, B, C, D, E, PP und deren Estern, den Carotinoiden, den Radikalenfängern, den Hydroxysäuren, den Antiseptika, den Molekülen, die auf Pigmentierung und auf Entzündung einwirken, den biologischen Extrakten, den Antibiotika, den Fungiziden, den Antiparasitenmitteln, den Malariamitteln, den Adsorptionsmitteln, den Hormonen und Hormonderivaten, Nikotin, den Antihistaminika, den steroiden und nicht steroiden Entzündungshemmern, den salicylsäurehaltigen Verbindungen, den Antiallergika, den Analgetika, den Lokalanästhetika, den antiviralen Mitteln, den Antikörpern und Molekülen, die auf das Immunsystem einwirken, wie Cyclosporin, Tacrolimus, den Hypolipidämika, wie Fibraten und Statinen, den Zytostatika und Krebsbekämpfungsmitteln, den Analgetika, den Vasodilatatoren, den Vasokonstriktoren, den Angiotensinkonversionsenzymhemmern und Phosphodiesterasehemmern, den Nitroderivaten und den Antianginosa, den Beta-Blockern, den Kalziumkanalblockern, den Antidiuretika und Diuretika, den Bronchodilatatoren, den Opiaten und Derivaten, den Barbituraten, den Benzodiazepinen, den Molekülen, die auf das zentrale Nervensystem einwirken, den Nukleinsäuren, den Peptiden, den Anthracenverbindungen, Paraffinöl, Polyethylenglycol, den Mineralsalzen, den Antispasmodika, den Magensäurehemmern, insbesondere die Klasse der Benzimidazole, darunter Omeprazol, Lansoprazol und Esomeprazol, und Ton und Polyvinylpyrrolidon enthaltende, die Magenwand schützende Medikamente, Stärke.

9. Verwendung eines galenischen Systems nach einem der Ansprüche 1 bis 8, **dadurch gekennzeichnet, dass** es bei einer Temperatur fest ist, die bis zu 45 °C, vorzugsweise bis zu 37,5 °C reichen kann.

10. Verwendung eines galenischen Systems nach einem der Ansprüche 1 bis 9, **dadurch gekennzeichnet, dass** die Lipidpartikel kugelförmig sind.

11. Verwendung eines galenischen Systems nach einem der Ansprüche 1 bis 10, **dadurch gekennzeichnet, dass** es die Form von Lipidpartikeln mit einer Größe im Bereich zwischen 0,5 Mikron und 1.500 Mikron, vorzugsweise zwischen 10 Mikron und 250 Mikron aufweist.

## Claims

1. Use of a galenic system for protecting an active substance against degradation during transit through the stomach when absorbed by oral route, **characterised in that** the galenic system in the form of strictly hydrophobic solid lipid particles containing absolutely no water, surfactants, emulsifiers or traces of solvents comprises at least one active ingredient, at least one hydrophobic wax and at least one agent neutralising the incorporated active substance; and **characterised in that** the neutralising agent is sodium bicarbonate as an alkalising agent, or an acidifying agent selected from among citric acid and tartaric acid.

2. Use of a galenic system according to claim 1, **characterised in that** the acidifying agent is in an amount of between 0.1 % and 20% by mass and preferably between 0.5% and 5%.

3. Use of a galenic system according to claim 1, **characterised in that** the sodium bicarbonate is in an amount of between 0.1 % and 30% by mass and preferably between 1% and 10%.

4. Use of a galenic system according to any one of claims 1 to 3, **characterised in that** the hydrophobic wax is a vegetable, animal or mineral wax, or a mixture of at least one wax and at least one non-amphiphilic oil.

5. Use of a galenic system according to any one of claims 1 to 4, **characterised in that** the wax has a melting point which may be between 15°C and 75°C, preferably between 30°C and 45°C.

6. Use of a galenic system according to any one of claims 1 to 5, **characterised in that** the wax is in an amount of between 0.5% and 99%, preferably between 1% and 55%.

7. Use of a galenic system according to any one of claims 1 to 6, **characterised in that** the loading capacity of the particles with active substance may range from 0.02% to 75% in relation to the weight of particles, particularly from 5% to 50%.

8. Use of a galenic system according to any one of claims 1 to 7, **characterised in that** the active substance is selected from among vitamins or provitamins A, B, C, D, E, PP and the esters thereof, carotenoids, anti-radical substances, hydroxy acids, antiseptics, molecules acting on pigmentation, on inflammation, biological extracts, antbiotics, antifungals, antiparasitics, antimalarials, adsorbents, hormones and hormone derivatives, nicotine, antihistamines, steroidal or non-steroidal anti-inflammatories, salicylate compounds, antiallergic agents, analgesics, local anaesthetics, antivirals, antibodies and molecules acting on the immune system such as cyclosporine, tacrolimus, hypolipidaemics such as fibrates and statins, cytostatics and anticancer agents, analgesics, vasodilators, vasoconstrictors, angiotensin converting enzyme inhibitors and phosphodiesterase inhibitors, nitrate derivatives and antianginal agents, beta-blockers, calcium inhibitors, antidiuretics and diuretics, bronchodilators, opiates and opiate derivatives, barbiturates, benzodiazepines, molecules acting on the central nervous system, nucleic acids, peptides, anthracene compounds, paraffin oil, polyethyleneglycol, mineral salts, antispasmodic agents, gastric antisecretory agents, particularly the class of benzimidazoles including omeprazole, lansoprazole and esomeprazole, clay gastric dressings and polyvinylpyrrolidone, starch.

9. Use of a galenic system according to any one of claims 1 to 8, **characterised in that** it is solid at a temperature of up to 45°C, preferably up to 37.5°C.

10. Use of a galenic system according to any one of claims 1 to 9, **characterised in that** the lipid particles are in spherical form.

11. Use of a galenic system according to any one of claims 1 to 10, **characterised in that** it is in the form of lipid particles between 0.5 microns and 1500 microns in size, preferably between 10 microns and 250 microns in size.
